# EUROPEAN PATENT SPECIFICATION

(11) **EP 2 131 829 B1**
(45) Date of publication and mention of the grant of the patent: **18.04.2012**
(21) Application number: 08717161.7
(22) Date of filing: 27.02.2008
(51) Int. Cl.: A61K 31/136, A61K 31/166, A61K 31/196, A61K 31/343, A61K 31/357, A61K 31/4439, A61K 31/47, A61K 31/606, A61K 31/609, G01N 33/94, A61P 1/00, A61P 1/02, A61P 1/04, A61P 11/02, A61P 17/00

(54) **PPAR-gamma agonists for the induction of cationic antimicrobial peptide expression as immunoprotective stimulants**
PPAR-gamma-Agonisten zur Einleitung der kationischen antimikrobiellen Peptid-Expression als immunprotektive Stimulantien
Agonistes de PPAR-gamma destinés à induire une expression peptidique antimicrobienne cationique servant de stimulants immunoprotecteurs

(30) Priority: 28.02.2007 IE 20070129
(43) Date of publication of application: 16.12.2009
(73) Proprietor: Giuliani International Limited, Dublin 2 (IE)
(72) Inventor: BARONI, Sergio, I-24030 Villa D'Adda (IT); DESREUMAUX, Pierre, F-59700 Marq En Baroeul (FR); BELLINVIA, Salvatore, I-33170 Pordenone (IT)
(74) Representative: Gates, Marie Christina Esther
(86) International application number: PCT/EP2008/052354
(87) International publication number: WO 2008/104557

(56) References cited:
- EP-A- 0 291 159
- EP-A- 0 352 826
- WO-A-98/06387
- WO-A-2004/073622
- WO-A-2007/010514
- WO-A-2007/010516
- US-B1- 6 326 364
- PEYRIN-BIROULET ET AL: "O02 PEROXISOME PROLIFERATOR-ACTIVATED RECEPTOR GAMMA FUNCTIONS AS AN ANTIBACTERIAL FACTOR" 20070301, vol. 1, no. 1, 1 March 2007 (2007-03-01), pages 2-3, XP022365961
- LIAO Y Z ET AL: "[Therapeutic effect of methyl 5-aminosalicylate on experimental ulcerative colitis in rabbits]" ZHONGGUO YAO LI XUE BAO = ACTA PHARMACOLOGICA SINICA JAN 1990, vol. 11, no. 1, January 1990 (1990-01), pages 54-56, XP008091453 ISSN: 0253-9756
- WANG T-T ET AL: "Cutting Edge:1,25-Dihydroxyvitamin D3 Is a Direct Inducer of Antimicrobial Peptide Gene Expression" JOURNAL OF IMMUNOLOGY, THE WILLIAMS AND WILKINS CO. BALTIMORE, vol. 73, 1 January 2004 (2004-01-01), pages 2909-2912, XP002992051 ISSN: 0022-1767 cited in the application
- SCHAUBER EIRGEN ET AL: "Histone-deacetylase inhibitors induce the cathelicidin LL-37 in gastrointestinal cells" MOLECULAR IMMUNOLOGY, vol. 41, no. 9, July 2004 (2004-07), pages 847-854, XP002480541 ISSN: 0161-5890
- SCHWAB ET AL: "Role of nuclear hormone receptors in butyrate-mediated up-regulation of the antimicrobial peptide cathelicidin in epithelial colorectal cells" MOLECULAR IMMUNOLOGY, ELMSFORD, NY, US, vol. 44, no. 8, 1 December 2006 (2006-12-01), pages 2107-2114, XP005792749 ISSN: 0161-5890
- ROUSSEAUX C ET AL: "Intestinal antiinflammatory effect of 5-aminosalicylic acid is dependent on peroxisome poliferator-activated receptor-gamma", THE JOURNAL OF EXPERIMENTAL MEDICINE, ROCKEFELLER UNIVERSITY PRESS, US, vol. 201, no. 8, 18 April 2005 (2005-04-18), pages 1205-1215, XP002414312, ISSN: 0022-1007, DOI: 10.1084/JEM.20041948

## Description

### Field of the Invention

The present invention relates to the induction of the defensins by PPAR-gamma agonists. Defensins provide immunoprotection to the skin, oral, nasal, ocular epithelia and other epithelia mucosae, including the vaginal mucosae. In particular, the invention is concerned with stimulation of defensins production by such agonists through activation of the PPAR receptors in epithelia and/or mucosae. More particularly, the invention is concerned with stimulation of enteric defensins production by such agonists by activation of the gut PPAR receptors.

### Background to the Invention

Inflammatory bowel diseases (IBD), including Crohn's disease (CD) and ulcerative colitis (UC), are chronic recurrent diseases with remissions and exacerbations, appearing principally in young patients. Inflammation may affect all regions of the bowel and all layers of the gut mucosa, including the adjacent mesenteric adipose tissue and perianal areas in CD. These diseases are clinically characterized by prolonged and variable courses, the diversity of intestinal manifestations, and by occurrence of serious local and systemic complications. The aetiology of CD and UC remains unknown, although the pathological intestinal inflammatory response is thought to be a consequence of a breakdown of tolerance to bacterial flora in the gastrointestinal tract of genetically predisposed individuals ¹.

IBD are more prominent in developed countries and particularly in Western Europe, North America, and Australia. Prevalence of CD and UC is about 1-2/1000 inhabitants. Notably in 2005, a total of 120 000 CD and 80 000 UC is estimated in France and about 2.5 million IBD patients in the United States. Without any available curative therapeutic molecules, the therapeutic management associates symptomatic therapy (analgesic, antibiotics, nutrition), anti-inflammatory and immunosuppressive agents (aminosalicylates, steroids, azathioprine, methotrexate, cyclosporine, monoclonal anti-TNF antibodies such as infliximab) and surgical treatment. Taken together, the development of new therapeutic molecules is therefore critical for the clinical management.

The digestive mucosa has evolved various immune strategies to tolerate intimate contact with commensals and prevent pathogenic bacteria from spreading into host tissues. The recognition of food-borne indigenous and pathogenic microbes is an essential barrier function for the survival of insects and mammals. In particular, mammalian resistance to pathogens is mainly conferred by membrane-bound Toll-like receptors (TLRs)¹ and the recently identified family of cytosolic nucleotide-binding oligomerization domain leucine-rich repeat containing proteins (NOD-LRRs)². NOD1 and NOD2 possess an N-terminal caspase recruitment domain (CARD), a central nucleotide-binding domain (NOD) and a C-terminal leucine-rich repeat domain (LRR)².

Considerable attention has been focused on NOD2 signalling, as mutations of this gene have been associated with Crohn's disease (CD)^{3,4}. NOD2 acts as a cytosolic pattern-recognition molecule (PRM) for bacterial peptidoglycan⁵ by detecting a major muropeptide released and recycled during bacterial growth - the muramyl dipeptide MurNAc-L-Ala-D-isoGln (MDP)⁶⁻⁸. Following recognition of MDP, NOD2 promotes and regulates both innate and adaptive immunity via transcriptional factors and kinase activation². Hence, the absence of NOD2 signalling in mice understandably confers oral susceptibility to Listeria monocytogenes via the regulation of certain enteric cationic antimicrobial peptides (CAMPs)⁸. Recent work has provided evidence that NOD1 confers responsiveness to peptidoglycans containing meso-diaminopimelic acid (primarily found in Gram negative bacteria)^{9,10}. Similarly to the physiological role of NOD2, NOD1 is required for expression of certain β-defensins by gastric epithelial cells during Helicobacter pylori infection¹¹.

### Gastrointestinal antimicrobial peptides (CAMPs: defensins, cathlelicidins): implications for inflammatory disease

Recent reports have shed light on the effector role of CAMPs in monitoring gut homeostasis and containing invading microbes, since the stem cells that replenish gut epithelium require continuous antimicrobial protection. The level of CAMP expression parallels intestinal development in metazoans, from the immaturity of local defense mechanisms during gestation to bacterial colonization of the gut after birth¹². Of particular interest are the NF-κB-dependent and independent regulation of two types of CAMP which are prevalent in the mammalian gut, namely the defensins and cathelicidins.

The α- and β-defensins are small polypeptides with spatially separated hydrophobic and positively charged residues. Six invariant cysteines form 3 specific intramolecular disulfide bonds and thus stabilize the protein in a complexly folded, triple-stranded beta-sheet configuration ^{12,13}. Whereas the α-defensins HD-1 to HD-4 (also known as human neutrophil proteins 1 to 4) are expressed by neutrophils, HD-5 and HD-6 (also known as cryptdins in mice) are produced by specialized intestinal epithelial cells, called Paneth cells. The latter are located primarily at the base of the crypts of Lieberkühn in the small intestine and have a major role in the innate immunity of the ileal mucosa by synthesizing and releasing proteinaceous granules into the lumen following exposure to microbes and/or microbial products. These secretory granules are rich in amphipathic peptides which can cause microbial death by disrupting membrane integrity¹²

Paneth cells play a crucial role in maintaining the tolerance towards commensals and in repelling pathogenic infections by producing antimicrobial peptides, such as defensin. The Wnt/Tcf/beta-catenin signaling pathway is essential in controlling intestinal homeostasis and Paneth cells differentiation.

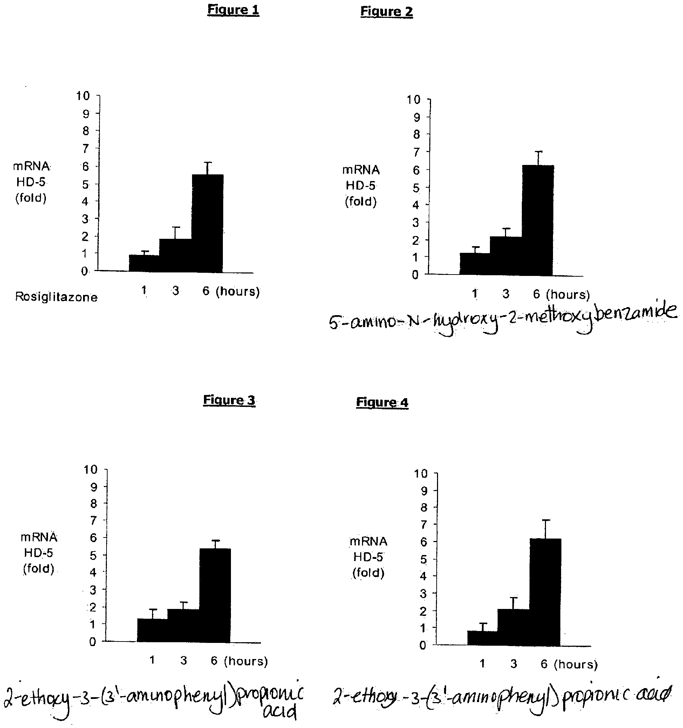

Interestingly, impaired intestinal expression of enteric alpha-defensins (namely HD-5) has been reported in CD which might contribute to changes in the luminal flora and/or generate vulnerability throughout the epithelial barrier to infection with enteropathogens ⁴, such as adherent-invasive E. coli⁵⁹ and M. paratuberculosis⁶⁰ (Figure 2). The influence of other microbial sensors and the CAMPs on the emergence of colonic disease also needs to be clarified, since the physiological bacterial load is higher in the colon than in the small intestine. Finally, the use of mice with mutant CAMPs and a recently developed mouse model carrying the major CD-associated NOD2 mutation⁶¹ may help determine if impaired enteric defensin function and/or natural NODs mutations are sufficient to trigger the development of intestinal inflammatory diseases. The in vivo antimicrobial function of α-defensins has been experimentally exemplified by observation of greater resistance to Salmonella typhimurium infection in HD-5 transgenic mice, accompanied by marked changes in the composition of the dominant flora in the gastrointestinal lumen¹⁴. Unlike β-defensins, the α-defensins produced by Paneth cells are mainly regulated at a post-transcriptional level by extracellular proteases¹⁵, including matrix metalloproteinase-7 (MMP-7, matrilysin) and trypsin in mice and humans, respectively^{16,17}. Hence, MMP-7^{-/-} mice accumulate inactive forms of cryptdins and succumb more readily to oral infection with S. typhimurium than do wild-type animals¹⁷. Six human β-defensins (hBD-1 to hBD-6) are primarily synthesized by most epithelial cells. Mice lacking the hBD1 orthologue show increased susceptibility to Staphylococcus aureus infection¹⁸, supporting a role for this protein in innate immunity.

Cathelicidins are CAMPs which are structurally and evolutionary distinct from defensins but which have a similar abundance and distribution in the gastrointestinal tract¹². They are synthesized as large precursor peptides containing a highly conserved N-terminal domain (cathelin), linked to a C-terminal peptide with antimicrobial activity. As with defensins, cathelicidins are activated by extracellular, partial proteolysis¹⁹. Although several members of the family have been identified in other mammalian species, humans and mice possess a single cathelicidin gene (referred as LL37/FALL39/hCAP18 and cathelin-related anti-microbial peptide (CRAMP), respectively)²⁰. Experimental evidence has indicated that mice lacking CRAMP are more susceptible to cutaneous infection by group A streptococci and urinary tract infection by invasive Escherichia coli^{21,22}. Furthermore, CRAMP-deficient macrophages failed to control replication of S. typhimurium²³.

Mice bearing mutations in the NF-κB and MyD88 signalling pathways display increased susceptibility to Helicobacter-induced colitis²⁴ and commensal-triggered colitis respectively²⁵, indicating an essential role for NF-κB in gut tolerance/resistance to bacteria and a potential involvement in the regulation of CAMP production. In humans, hBD-1 expression is constitutive in the small intestine and the colon, whereas colonic synthesis of hBD-2 to -4 is strongly dependent on NF-κB activation by infectious agents in the digestive tract (such as H. pylori) and/or pro-inflammatory cytokines¹². In addition to the regulatory impact of TLR signalling²⁶, the NOD1 and NOD2 signalling pathways have been shown to trigger hBD-2 expression^{11,27}. Furthermore, recent findings have shown that activation of the MAP kinase pathways is also required for hBD-2 and/or -3 expression^{11,26}.

It has been shown that three mutations in the NOD2 gene (namely R702W, G908R and 1007fs) lead to a predisposition to CD^{3,4}. Genotype-phenotype correlations have established that NOD2 mutants are predominantly linked to ileal CD⁴². Both common and rare mutations have been associated with impaired MDP-induced NF-κB activation^{5,7} and cytokine production in peripheral blood monocytes^{7,43-45}. Lala and collaborators recently reported that NOD2 is highly expressed in Paneth cells^{46,47}, a finding which might account for the association between NOD2 mutations and the development of ileal inflammatory lesions⁴². In agreement with a protective effect of NOD2 in the ileum, Nod2-knockout mice displayed (i) enhanced susceptibility to oral infection (but not systemic infection) with the Gram positive facultative intracellular bacterium L. monocytogenes and (ii) markedly decreased expression of a subgroup of cryptdin genes⁸.

Importantly, decreased production of HD-5 and HD-6 has been found in surgical resection specimens and biopsies from ileal CD patients^{14,48,49}; reportedly, the CD-associated NOD2 mutations contributed to this impairment. On the other hand, individuals with Crohn's colitis displayed normal α-defensin levels but have a much reduced copy number for the β-defensin gene hBD-2, resulting in impaired expression in the colon⁵⁰, As with NOD2 mutations, complex intronic polymorphism of the NOD1 gene has been associated with the pathogenesis of IBD⁵¹. In addition, Nodi-deficient mice display increased susceptibility to H. pylori infection⁵² and decreased expression of certain β-defensins¹¹.

Reduced expression of defensins in ileal CD might contribute to changes in the luminal flora, thus generating vulnerability throughout the epithelial barrier to infection with CD-associated pathogens such as adherent-invasive E. coli⁵⁹ and M. paratuberculosis⁶⁰

In parallel, NF-κB-independent signalling pathways might control CAMP production by regulating epithelium cell renewal, differentiation and/or lineage commitment. Interestingly, impaired Wingless (Wnt) signalling is associated with a complete lack of proliferative cells in the foetal small intestinal epithelium²⁸, suggesting that this pathway has an essential role in maintaining the proliferative/undifferentiated status of intestinal epithelial cells. The absence of the ephrinB3 gene (which is downregulated by the Wnt signalling pathway) was seen to result in abnormal Paneth cell lineage commitment²⁹. Moreover, the Wnt signalling pathway might monitor defensin gene expression (via the transcription factor 4, Tcf4) in cells derived from Paneth cells, since cryptdins were not detected in the small intestine of embryonic Tcf4-/- mice or that of adults lacking the Wnt receptor Frizzled-5³⁰. Conversely, cryptdin genes are overexpressed in mice showing mutational activation of the Wnt signalling pathway^{30,31}.

A site-directed mutational analysis of α-defensin promoters revealed an essential, regulatory role for TCF binding sites³⁰. Taken as a whole, these findings indicate that activation of the Wnt signalling is required for the production of Paneth cell-derived CAMPs. Hence, Paneth cell determinants (such as Mtgr1 and Gfi1) should be considered as potential candidates for susceptibility to chronic inflammatory disorders^{32,33}.

Finally, and given the crucial role of certain nuclear receptors in immunity, bacterial-induced inflammation and cell proliferation/maturation, it has been suggested that these receptors may have a potential role in gastrointestinal innate immunity by regulating CAMP biogenesis. Interestingly, a glucocorticoid receptor agonist (dexamethasone) has been shown to enhance hBD-2 expression, although the mechanism remains to be determined³⁴. More recently, cathelicidin- and hBD2-encoding genes were identified as targets of the vitamin D receptor (VDR)³⁵, a nuclear receptor required for resistance to M. bovis infection³⁶. Treatment of monocytes with a synthetic VDR ligand led to dose-dependent up-regulation of cathelicidin gene transcription, which exerts a direct antimicrobial effect on M. tuberculosis³⁷. In agreement with these findings, individuals with decreased endogenous VDR ligand levels display increased susceptibility to M. tuberculosis infection³⁷.

To circumvent the CAMPs' microbicidal activity, microorganisms (generally pathogens) have developed a range of strategies which are reminiscent of those involved in antibiotic resistance³⁹. One way to achieve inactivation is to produce proteases, which degrade CAMPs; however, in the case of defensins, the intramolecular disulphide bridges render the peptides relatively resistant to enzymatic proteolysis. Another stratagem consists in reducing the net cationic charge of the bacterial envelope, in order to lower its affinity for CAMPs; this is achieved by incorporating positively-charged groups into the teichoic acid polymers (D-alanine) and in the lipid A (aminoarabinose) in the bacterial cell wall. Other bacterial approaches to CAMP resistance include preventing the host effectors from accessing their target via extracellular capture by secretory proteins and actively pumping the peptides across the cytoplasmic membrane³⁹. However, despite these various protective weapons (which are not mutually exdusive), microorganisms will probably still be inhibited by CAMPs if the host is capable of releasing the latter in high amounts into the intestinal lumen, as in the case of defensins. In such a situation, down-regulation of CAMP-encoding genes at the transcriptional level by bacterial components (as reported in patients with shigellosis⁴⁰ and in mice orally infected with S. typhimurium⁴¹) may be a very sophisticated counter-mechanism (Figure 2).

Impaired microbial sensing and aggression by specific enteric microbes may affect CAMPs function in the gut and result in the development of chronic inflammatory disorders, such as inflammatory bowel disease (IBD). These findings shed new light on the pathogenesis of CD, which is classically viewed as resulting from an abnormal T-cell activation by microbial immungens.

In addition to the antimicrobial activity of CAMPs, pleitotropic functions have been attributed to defensins and cathelicidins (Table 1)⁵³. Both CAMPs have the ability to chemoattract immunocytes involved in innate immunity (neutrophils and monocytes/macrophages), adaptive immunity (dendritic cells and T lymphocytes) and allergic/inflammatory reactions (mast cells). Furthermore, hBD2 might activate the TLR4-dependent signalling pathway in dendritic cells⁵⁴.

On the other hand, Hancock et al. recently reported that LL-37 may dampen TLR-dependent activation in human monocytes⁵⁵ and may promote maturation of dendritic cells, resulting in Th1 polarization of T cells⁵⁶. Taken as a whole, these findings indicate that CAMP interactions can initiate and control the inflammatory response by linking innate and acquired immunity (Table 1). Finally, CAMPs such as LL-37 have the ability to promote angiogenesis, as demonstrated by decreased vascularization during skin wound repair in CRAMP-deficient mice⁵⁷. Since Paneth cell biology influences intestinal angiogenesis through the recognition of commensals⁵⁸, these findings provide insight into the pathogenesis of IBD. Enteric CAMPs have a number of essential and emerging roles in both innate and adaptive immunity of the gastrointestinal tract by modulating microbial resistance, angiogenesis, chemotaxis and the activation/maturation of the humoral response (Table 1). In particular, release of CAMPs into the lumen is thought to protect the mitotically-active crypt cells (which renew the epithelial cell monolayer) from colonization by pathogenic microbes. The use of transgenic animals yield a better understanding of the physiological role and regulation of these effectors. NOD1/2 have been shown to exert bactericidal activity by modulating the epithelial production of defensins - suggesting a possible mechanism whereby PRMs Pattern Recognition Molecules might protect the host from the development of CD (Figure 2).

### Epithelial antimicrobial peptides: implications for immune system stimulation

Several recent studies have implicated antimicrobial peptides, including defensins, in protective roles in skin, oral, nasal, ocular epithelia and other epithelia mucosae, including vaginal mucosae. All mucosae share a common embryogenetic origin, since all originate from the same embroyonic layer and might be expected to exhibit similar biochemical responses, including protective defensin expression.

In 2003, Dinulos et al. examined the antimicrobial activity of keratinocyte expression of β-defensin-2 in cutaneous immune defenses. β-defensin-2 expression was found to be induced by Staphylococcus aureus, Streptococcus epidermidis, Escherichia coli, and Pseudomonas aeruginosa, whereas Streptococcus pyogenes was found to be a poor β-defensin-2 inducer. The study indicated that the ability to induce β-defensin-2 expression in combination with it antimicrobial effects may contribute to the rarity of skin infections with gram-negative bacterial organism whereas the lack of stimulation afforded by Streptococcus pyogenes may point towards its ability to evade immune system defences and cause skin disease.⁶⁷

In the past year, Huang et al. have carried out antimicrobial assays to investigate the antimicrobial activity of a number of human ocular surface expressed antimicrobial peptides, including β-defensins 1 - 3, against microbes such as Pseudomonas aerginosa (PA), Staphylococcus aureus (SA) and Staphylococcus epidermidis (SE) in the presence of NaCl or tears. β-defensin-3 was shown to have potent activity against both SA and SE, whereas β-defensin-2 had moderate activity and β-defensin-1 showed no activity against these strains. Activity was attenuated by NaCl and tears completely inhibited the activity of β-defensin-1 and 2, but did not affect the β-defensin-3 activity. The study validates the role of some defensins as an in vivo antimicrobial.⁶⁸

Towards late 2007, Vanhinsbergh showed that reduced levels of certain immunomodulatory gene expression, particularly toll-like receptors (TRLs) and defensins, are associated with allergy development, for example allergic and nonallergic rhinitis development.⁶⁹

Finally, Chung et al have identified specific signaling routes that pathogens and commensals take in stimulating antimicrobial peptides such as defensins and cathelicidins in skin, oral mucosal and other epithelia, and identify these routes with development of new therapeutic agents for periodontal diseases.⁷⁰

Such work reinforces the protective role of defensins in the human immune system and highlights the importance of increasing immune response to pathogens by providing routes to stimulation of defensin production in the body.

### Other Background Art

United States Patent No: US 6,326,364 describes 5-aminosalicylate compounds such as 5-ASA as having selective antimicrobial effects in vitro. Examples show inhibitory effects against a series of Clostridium bacterial cultures on agar plates in aerobic and anaerobic conditions. No effect was observed against colonies of Lactobacillus Enterococcus or Bacteroides.

International Publication No's. WO2007/010514 and WO2007/010516 describe the use of a number of 5-ASA structural analogues which function as PPAR agonists and can be used in treatment of inflammatory gastrointestinal conditions and cancer.

### PPAR-gamma Roles

Recently, the nuclear receptor peroxisome proliferator-activated receptor gamma (PPAR-gamma) was identified as a target of anti-inflammatory drugs used in the treatment of IBD, indicating a mechanism by which they mediate in vivo anti-inflammatory effect in the gut ², However, presumably due to the common mucosae embryogenetic origin, PPAR receptor expression has been demonstrated in various mucosal zones, other than the gut. Recent studies have suggested that PPAR-gamma may contribute to chronic inflammation of the nasal muscosa in perennial allergic rhinitis⁷⁰.

PPAR-gamma is an essential nuclear receptor controlling intestinal homeostasis by interacting with beta-catenin and T cell transcription factor (Tcf-4). Tcf-4 is an essential transcription factor in determining intestinal cell fate and in regulating the expression of natural antibiotics by Paneth cells³.

5-aminosalycilate (5-ASA) is an anti-inflammatory drug (mesalazine) widely used in the treatment of inflammatory bowel diseases, but the mechanism underlying its intestinal effects remained poorly understood. Recently, the nuclear receptor peroxisome proliferator-activated receptor gamma (PPAR-gamma as identified as a target of 5-ASA, indicating a mechanism by which 5-ASA mediate in vivo its anti-inflammatory effect in the gut ². Hence given the antimicrobial properties of 5-ASA, such compounds and its derivatives might modulate the expression of antimicrobial genes through PPAR-Y activation. Rosiglitazone also effects activation of the peroxisome proliferator-activated receptors (PPARs), specifically PPAR-gamma, it has an anti inflammatory effect.

### Definitions

To facilitate understanding of the invention, a number of terms are defined below.

As used herein, the term "antimicrobial peptides" refers to either α- or β-defensins (e.g. HD-5 for human defensin 5), or small polypeptides with spatially separated hydrophobic and positively charged residues.

As used herein, the term "activates defensins", when used in reference to any molecule that activates defensins, refers to a molecule (i.e., a PPAR-gamma agonist) that induces the gene expression of α- or β-defensins.

As used herein, the term "primer" refers to a synthetic oligonucleotide, which is capable of acting as a point of initiation of synthesis when placed under conditions in which synthesis of a primer extension product which is complementary to a nucleic acid strand is induced.

As used herein, the term "polymerase chain reaction" (hereinafter "PCR") is a method for amplifying a segment of a target sequence in a mixture of genomic DNA whithout cloning or purification. The two primers are complementary to their respective strands of the double stranded target sequence.

As used herein, the term "structural analogues" relate to compounds whose molecular structure act as mimics of 5-ASA in respect of their binding ability to the PPAR-gamma receptor. In particular; those compounds whose structures allow for analogous types of hydrogen bonding, and electrostatic interactions at the PPAR-gamma receptor.

As used herein, the term "Caco-2 cells" refers to human Caucasian colon adenocarcinoma cells.

As used herein, the term "rosiglitazone" refers to a highly selective and potent chemical agonist for PPAR-gamma. As used herein, the term "epithelia" refers to body tissues composed of layers of cells that cover organ surfaces such as surface of the skin and inner lining of digestive tract.

As used herein, the term "muscosae" refers to the mucous membranes are linings of mostly endodermal origin, covered in epithelium, which are involved in absorption and secretion. Muscosae line various body cavities that are exposed to the external environment and internal organs. They are found continuous with skin at the nostrils, the lips, the ears, the genital area, and the anus.

### Object of the Invention

The scope of the invention is defined by the claims. Also disclosed are PPAR-gamma agonists which can stimulate PPAR-gamma receptors to induce enteric defensin expression in the gut.

Also disclosed are PPAR-gamma agonists, which comprise a series of compounds found to be active on the PPAR receptor for use as stimulants to induce enteric CAMP expression in the gut, particularly defensin expression.

Also disclosed are compounds that are capable of killing microbes by stimulating CAMP expression. CAMPs may be defensin and/or cathelicidin. Microbes are agents that are capable of killing bacterial, viruses, fungi and other infectious agents.

Also disclosed are compounds that enhance the body's defence mechanisms through expression of CAMP. CAMP may be defensin and/or cathelicidin. The object of the present invention is to provide compounds for use in the intervention of gastrointestinal tract condition acute diverticulitis.

It is a preferred object of the invention to provide an intervention for the prevention of acute diverticulitis in patients affected by colonic diverticulosis, indeterminate colitis and infectious colitis.

It is a further object of the invention to provide compounds for use in preparation of a medicament for the treatment and prevention of such diseases.

Also disclosed are modulators of CAMP expression.

### Summary of the invention:

Disclosed is a method which uses a range of chemical entities which act on PPAR-gamma, to induce CAMP production. Such compounds can be used in the induction of CAMP expression in tissues having PPAR-gamma receptors, such as epithelia and/or mucosae. In particular, the compounds can be used to induce enteric CAMP expression in the gut. Defensins are examples of CAMPs.

Also disclosed are PPAR-gamma agonists which comprise a series of compounds found to be active on the PPAR-gamma receptor; for use as stimulants to induce CAMP expression in tissue, particularly defensin expression.

Also disclosed are PPAR-gamma agonists which comprise a series of compounds found to be active on the PPAR-gamma receptor, for use as stimulants to induce CAMP expression in epithelia and/or mucosae having PPAR-gamma receptors, particularly defensin expression.

Also disclosed are PPAR-gamma agonists which comprise a series of compounds found to be active on the PPAR-gamma receptor, for use as stimulants to induce enteric CAMP expression in the gut, particularly defensin expression.

The compounds of the invention may be selected from 2-methoxy-3-(4'-aminophenyl) propionic acid, 5-amino-N-hydroxy-2-methoxybenzamide, 2-ethoxy-3-(4'-aminophenyl) propionic acid or 2-ethoxy-3-(3'aminophenyl) propionic acid for use in the treatment and for prevention of acute diverticulitis.

The compounds can be used in the induction of CAMP expression in tissues having PPAR-gamma receptors, such as epithelia and/or mucosae. In particular, the compounds can be used to induce enteric CAMP expression in the gut.

The compounds of the invention can be selected from the group comprising: 3-(4'-aminophenyl)2-methoxypropionic acid "**R34**" 3-(4'-aminophenyl)2-ethoxypropionic acid 3-(3'-aminophenyl)2-ethoxypropionic acid.

The above compound names can also be written in standard chemical nomenclature as follows (which nomenclature will be used throughout the text): (±)-2-methoxy-3-(4'-aminophenyl) propionic acid "**R34**" **(racemic form)** (±)-2-ethoxy-3-(4'-aminophenyl) propionic acid (±)-2-ethoxy-3-(3'-aminophenyl) propionic acid.

In particular, the compounds for use in the methods of the present invention can be enantiomers of the following racemic mixtures: (R,S)-2-methoxy-3-(4-aminophenyl)propionic acid(compound 34) "**R34**" (**racemic form**) Enantiomers of R34:
(+) 2-S-methoxy-3'(4'-aminophenyl)propionic acid "**34-E1**" or "**E-1**".
(-) 2-R-methoxy-3-(4'-aminophenyl)propionic acid "**34-E2**" or "**E-2**"

Racemic mixtures of the compounds may also be for use in the methods described herein. Examples of racemic mixtures include. (±)-2-methoxy-3-(4'-aminophenyl)propionic acid "**R34**" (**racemic form**).

Compositions containing an excess of one enantiomer over another, for any of the stereoisomeric compounds described herein, may also be for use in the methods described herein.

According to another embodiment still, the compounds which may be used include the compound of formula (I)

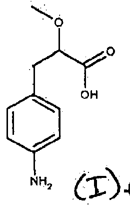

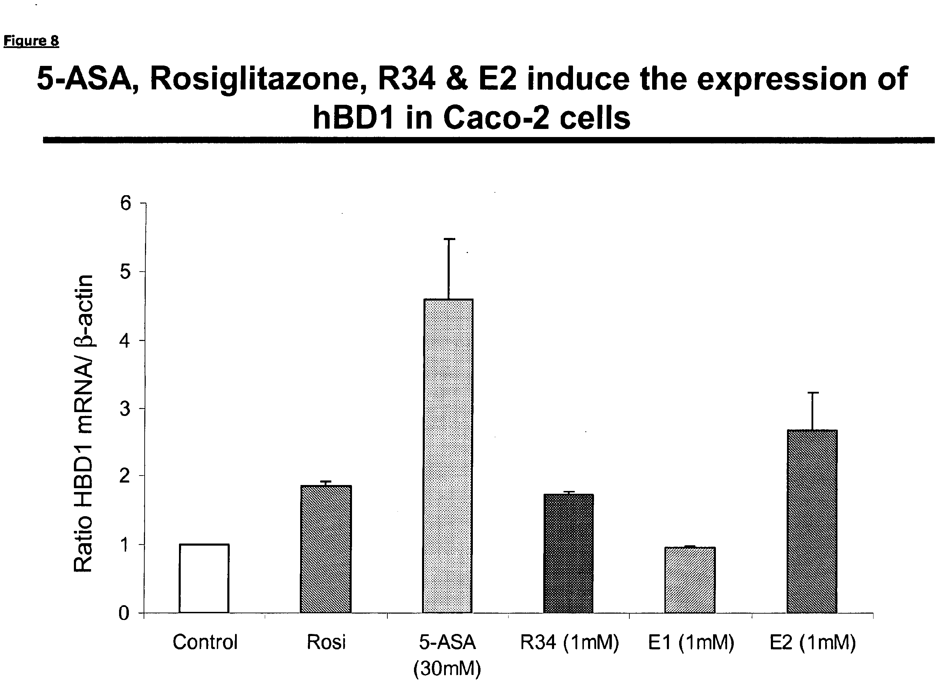

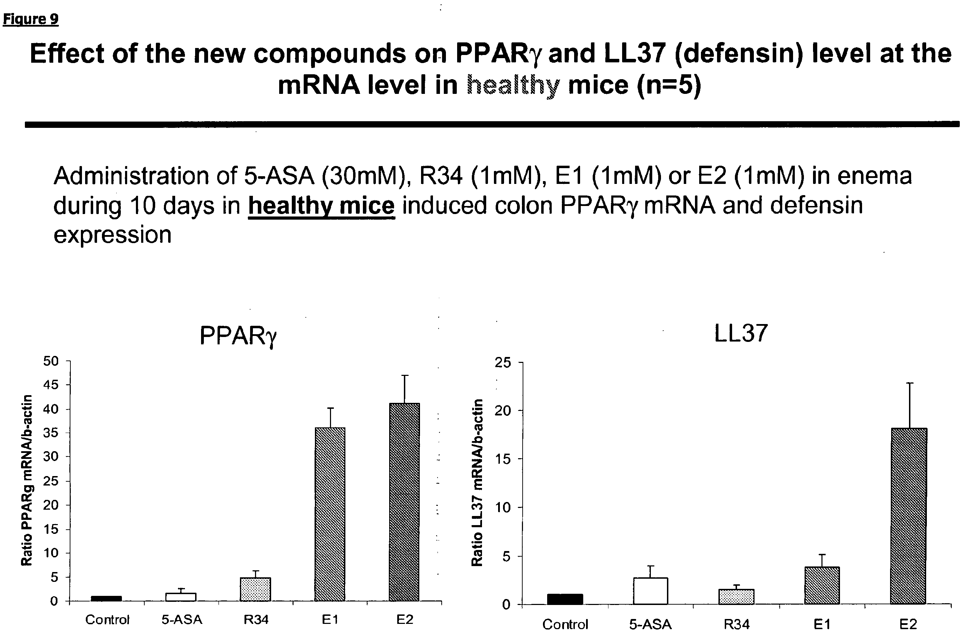

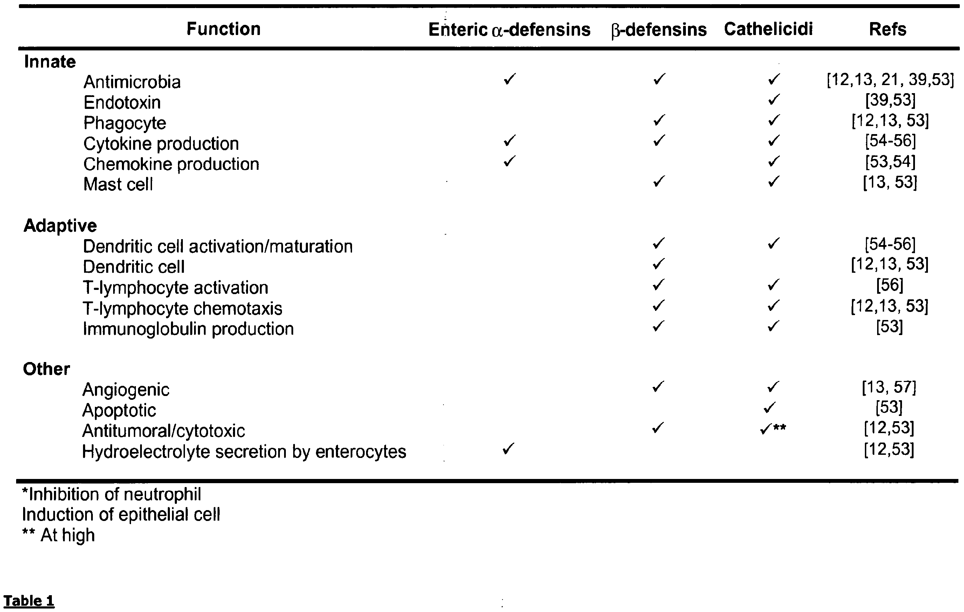

According to the invention, one enantiomer of (R,S)-2-methoxy-3-(4'-aminophenyl)propionic acid having formula (I) namely, (-) 2-R-methoxy-3-(4'-aminophenyl)propionic acid "**34-E2**" or "**E-2**", has been found to be particularly effective in gut defensin expression induction (Figures 8 & 9). According to the present invention some enantiomers may provide superior CAMP expression than their stereoisomers. In other embodiments, some racemic mixtures may provide superior CAMP expression than their individual stereoisomers.

According to another embodiment still, the compounds which may be used include the compound of formula (II)

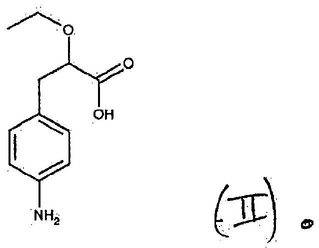

According to another embodiment still, the compounds which may be used include the compound of formula (III)

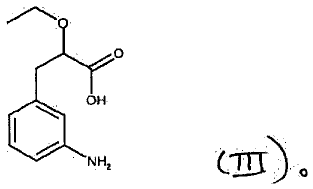

Preferably, the compounds which may be for use in the methods of the invention can be selected from the group comprising: (±)-2-methoxy-3-(4'-aminophenyl) propionic acid "**R34**"
(+) 2-S-methoxy-3-(3-aminophenyl)propionic acid "**34-E1**" **or** "**E-1**"
(-) 2-R-methoxy-3-(3-aminophenyl)propionic acid "**34-E2**" or "**E-2**"
(±)-2-ethoxy-3-(4'-aminophenyl) propionic acid
(±)-2-ethoxy-3-(3'-aminophenyl) propionic acid, whose formulas are shown earlier.

In particular, the compound 5-amino-N-hydroxy-2-methoxybenzamide can also be for use in the methods of the invention.

One example comprises use of the following compound :

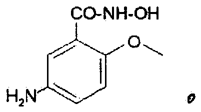

The present invention also relates to compounds for use in methods of treatment of humans and/or mammals (including rodents, farm animals, domestic pets, mice, rats, hamsters, rabbits, dogs, cats, pigs, sheep, cows, horses).

According to the present invention there are provided compounds for use in the intervention of the gastrointestinal tract condition acute diverticulitis.

In one aspect of the invention, there are provided compounds for use in the prevention of acute diverticulitis in patients affected by colonic diverticulosis, indeterminate colitis and infectious colitis.

The compounds according to the present invention can be used advantageously in the medical field to stimulate PPAR-gamma to produce CAMPs. CAMPS include defensin and/or cathelicidin. Therefore another aspect of the present invention relates to a pharmaceutical composition comprising one or more compounds as defined above as active principles in combination with one or more pharmaceutically acceptable excipients or adjuvants.

In another aspect, the invention provides compounds for use in preparation of a medicament for the treatment and prevention of acute diverticulitis and prevention of acute diverticulitis in patients affected by colonic diverticulosis, Indeterminate colitis and infectious colitis.

The present invention also relates to compounds for use in methods of treatment of humans and/or mammals (including rodents, farm animals, domestic pets, mice, rats, hamsters, rabbits, dogs, cats, pigs, sheep, cows, horses).

### Brief Description of the Drawings

**Figure 1****:** Rosiglitazone (reference compound) activates HD-5 in Caco-2 cells, as determined by quantitative PCR analysis.
**Figure 2****:** 5-amino-N-hydroxy-2-methoxybenzamide activates HD-5 in Caco=2 cells, as determined by quantitative PCR analysis.
**Figure 3****:** 2-ethoxy-3-(3'aminophenyl) propionic acid activates HD-5 in Caco-2 cells, as determined by quantitative PCR analysis.
**Figure 4****:** 2-ethoxy-3-(4'aminophenyl) propionic acid activates HD-5 in Caco-2 cells, as determined by quantitative PCR analysis.
**Figure 5****:** Mesalazine (reference compound) activates HD-5 in Caco-2 cells, as determined by quantitative PCR analysis.

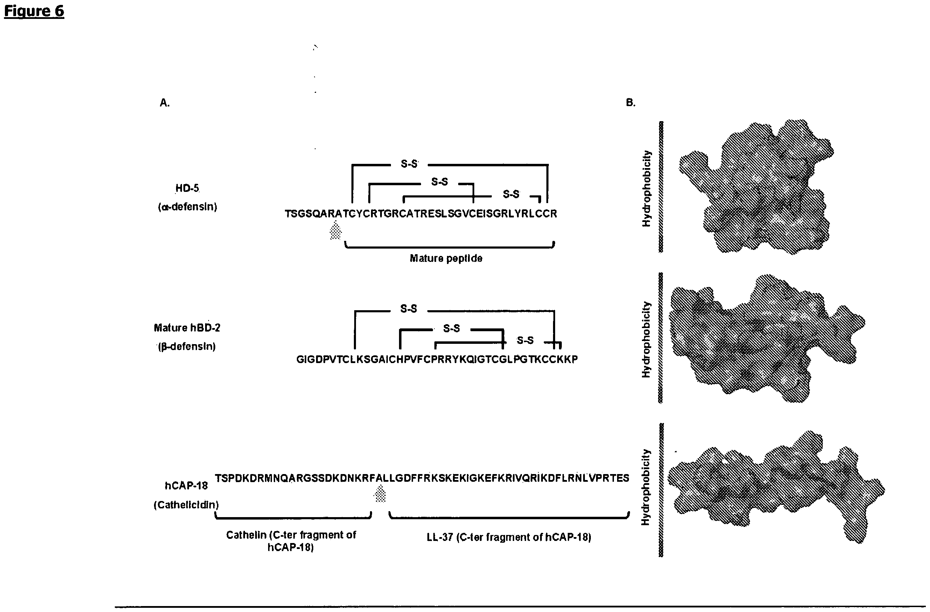

**Figure 6****: Structure of human defensins and cathelicidin**
   **A**. Human sequences of the enteric α-defensin HD-5, the β-defensin HBD-2 and the cathelicidin hCAP-18. Gray arrows depict the cleavage site for HD-5 and cathelicidin. Patterns of the three intramolecular disulfide bonds (S-S) of the α and β-defensins, have been notified in both the schematic and tri-dimensional structures (blue sticks).
   **B**. Athree-dimensional solution (h-BD2 and cathelicidin) or crystal (HD-5) structure of defensins and cathecidin is displayed. The Protein Data Bank accession numbers used for the illustration are the following: 1ZMP for HD-5, 1E4Q for h-BD2 and 2FCG for LL-37 (C-terminal fragment of hCAP-18). The beta turns are depicted in orange and the alpha-helices in red. The hydrophobicity of the molecules is displayed.

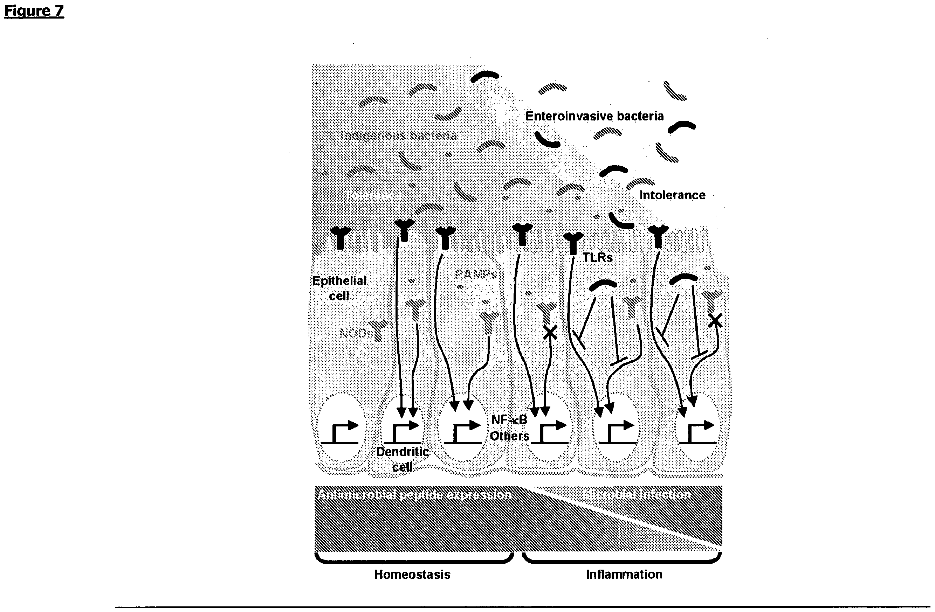

**Figure 7****: A pathophysiological model for chronic intestinal inflammation.** Once microbes and/or their products are sensed by TLRs and/or NODs (left side of the figure), CAMPs are synthesized via the action of NF-κB and/or other transcription factors (cf. the main text). Following their secretion and extracellular processing, the CAMPs (i) promote tolerance and the recruitment of inflammatory cells, (ii) prevent invasion of microbial pathogens and (iii) protect against the development of chronic intestinal inflammation. Abnormal antimicrobial peptide synthesis and/or function might lead to aberrant activation of the adaptive immune system and to intestinal inflammation (right side of the figure) by microbial threats and/or impaired innate immunity (i.e. NOD2 mutations),
**Figure 8****:** 5-ASA 1 (reference compound) , Rosiglitazone 2 (reference compound) racemic R34 & enantiomer 34-E2 induce the expression of hBD1 (human defensins-1) in Caco-2 cells.
**Figure 9**: Effect of racemic R34 & enantionmes 34-E1 & 34-E2 on PPAR-gamma and LL37 (defensin) level at the mRNA level in healthy mice (n=5). Administration of-S-ASA (30mM) (reference compound), R34 (1mM), E1 and E2 (1mM) by enema during 10 days in healthy mice induced colon PPARy mRNA and defensin expression.

**Table 1. Versatile functions of the defensins and cathelicidins**. The functions of α/β-defensins and cathelicidins are listed in the Table and discussed in the main text.

### Detailed Description of the Invention.

Given the direct negative role of the peroxisome proliferator-activated receptor gamma (PPAR-gamma) on the Wnt/Tcf/beta-catenin signaling pathway, PPAR-gamma activation was examined to investigate defensin biogenesis.

### Experimental Data

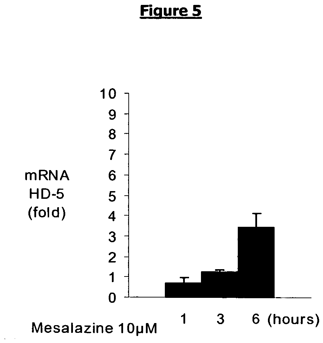

The tested PPAR--gamma agonists, rosiglitazone (at 1 µM for 1, 3 or 6 hours) and others, activates Hod-5 in Caco-2 cells, as determined by quantitative PCR analysis (Figures 1 - 5).

### Methods

Cultured intestinal epithelial cell lines, namely Cacao-2 (of human origin) and ICcl2 (of mice origin), were treated with the GSK-3 inhibitor LiCl (20 microM) or the phosphatase inhibitor calyculin (50 nM) followed or not by stimulation with the PPAR-gamma agonist such as rosiglitazone (1microM) (reference compound).

The expression of defensin and known target genes of both Wnt and PPAR-gamma signaling pathways were investigated by quantitative real-time PCR. The activation of GSK3, beta-catenin, NF-kappaB, ERK1/2, SAPK/JNK and p38 was measured by specific immunoblotting.

To investigate the antimicrobial role of PPAR-gamma, the intracellular replication of the Crohn's disease associated Escherichia coli (LF82) was measured upon or not stimulation with rosiglitazone in the Raw macrophage cell line.

### Results

Incubation with rosiglitazone and other PPAR-gamma activators significantly increased the expression of both alpha- (HD-5 and HD-6) and beta- (Defb10) defensins by intestinal epithelial cells. Such antimicrobial gene expression was synergized following co-stimulation by calyculin that promotes beta-catenin degradation. Accordingly, reduced intracellular replication of LF82 through PPAR-gamma activation by rosiglitazone was observed.

Conversely, the expression of a Wnt/Tcf/beta-Catenin target gene, cyclin-D1, and the stability of the beta-catenin was markedly decreased upon stimulation by both calyculin and rosiglitazone.

Finally, LiCl, an activator of the Wnt/TCF/beta-catenin-dependent signalling pathway, blocked the rosiglitazone-induced defensin gene expression upon co-stimulation.

Incubation with test substances significantly increased the expression of both alpha- (HD-5 and HD-6) and beta- (Defb10) defensins by intestinal epithelial cells . Accordingly, reduced intracellular replication of LF82 through PPAR-gamma activation by rosiglitazone was observed.

### Conclusion

Taken as a whole, the results indicate that PPAR-gamma activation promotes the induction of an antimicrobial gene programme by negatively regulating the formation of the Tcf/beta-catenin complex. These findings highlight the therapeutical potential of PPAR-gamma in complementing defensins deficiency in many gasterointestinal disorders such as Crohn's disease, ulcerative colitis; intestinal bowel syndrome, acute diverticulitis and for the prevention of condition such as acute diverticulitis in patients affected by colonic diverticulosis, indeterminate colitis and infectious colitis.

Furthermore, these findings highlight the therapeutical potential of PPAR gamma agonists in complementing defensins deficiency in other mucosal disorders including but not limited to those such as ocular inflammation and infections, periodontal disease, allergic and non allergic rhinitis, bacterial vaginosis and skin inflammatory conditions and infections such as impetigo, erysipela, dermatitis, folliculitis, acne and vulgaris.

### In vitro studies with racemic compound 34 and enantiomers 34-E1 & 34-E2 Materials

5-ASA 1 (reference compound) was purchased at Sigma-Aldrich (St Quentin Fallavier, France). Rosiglitazone 2 (reference compound) was synthesized in the laboratory according to standard procedures. The racemic compound 34 and the two enantiomers of the compound, 34-E1 and 34-E2 were provided by Giuliani SpA (Milano, Italy). Compound were re-suspended in DMEM medium (Gibco) and adjusted at pH=7 if necessary with 10N NaOH.

### Regulation of the expression of hBD1 defensin in colonic epithelial cells Cell lines

The colon carcinoma cell line Caco-2 (ATCC HTB-39) was routinely grown in DMEM supplemented respectively with 10% or 20% heat-FCS, and antibiotics. Cells were grown in monolayers, incubated at 37°C in 5% CO2 and 95% relative humidity.

Cell were stimulated by 5-ASA, R34, 34-E1 and 34-E2 for 24h. Total RNA was isolated from cells using Rneasy kit (Macherey Nagel, Hoerdt, France) according to the manufacture's instructions. RNA quantification was performed using spectrophotometry. After treatment at 37°C for 30 min with 20-50 units of RNase-free DNase I (Roche Diagnostics Corporation, Indianapolis, IN, USA), oligo-dT primers (Roche Diagnostics Corporation, Indianapolis, USA) were used to synthesize single-stranded cDNA. mRNAs were quantified using SYBR green Master Mix (Applera, Courtaboeuf, France) with human specific oligonucleotides for hBD1(S:5'-ATACTTCAAAAGCAATTTTCCTTTAT-3'; AS:5'-TTgTCTGAGATGGCCTCAggTggTAAC-3') in a GeneAmp Abiprism 7000 (Applera, Courtaboeuf, France). In each assay, calibrated and no-template controls were included. Each sample was run in triplicate. SYBR green dye intensity was analyzed using the Abiprism 7000 SDS software (Applera, Courtaboeuf, France). All results were normalized to the unaffected housekeeping gene of human β-actin (S:5'-TCACCCACACTgTgCCCATCTACg-3'; AS:5'-CAgCggAACCgCTCATTgCCAATg-3').

### Evaluation of β-defensin expression in healthy mice

5-ASA (30mM), racemic R34 and 34-E1 & 34-E2 (1mM) were administrated by intrarectal instillations for 8 days. Post-mortem, total RNA was isolated from whole mice colonic tissues using Rneasy kit (Macherey Nagel, Hoerdt, France) according to the manufacturer's instructions. RNA quantification was performed using spectrophotometry. After treatment at 37°C for 30 min with 20-50 units of RNase-free DNase I (Roche Diagnostics Corporation, Indianapolis, IN, USA), oligo-dT primers (Roche Diagnostics Corporation, Indianapolis, USA) were used to synthesize single-stranded cDNA. mRNAs were quantified using SYBR green Master Mix (Applera, Courtaboeuf, France) with mouse specific oligonucleotides for LL37(S:5'-gCTgATTCTTTTgACATCAgCTgTAA-3' AS:5'-gCCAgCCgggAAATTTTCT-3') in a GeneAmp Abiprism 7000 (Applera, Courtaboeuf, France). In each assay, calibrated and no-template controls were included. Each sample was run in triplicate. SYBR green dye intensity was analyzed using the Abiprism 7000 SDS software (Applera, Courtaboeuf, France). All results were normalized to the unaffected housekeeping gene β-actin (S:5'-gggTCAgAAggATTCCTATg-3'; A:5' ggTCTCAAACATgATCTggg-3').

### In vivo study

### Regulation of visceral pain in rats

### Animals

Male Sprague-Dawley rats (Charles River, l'Arbresle, France) weighing 175-200 g were used in this study. Rats were maintained in laboratory conditions for 1 week before experiment. The animals were housed 5 per cage with food and water available ad libitum. All studies were performed in accordance with the proposal of the committee for Research and Ethical Issues of the International Association for the Study of Pain (Zimmermann M, Pain 1983; 16:109-110). Great care was taken, particularly with regard to housing conditions, to avoid or minimize discomfort to the animals.

### Evaluation of colonic sensitivity

Nociception in the animals was assessed by measuring the intracolonic pressure required to induce a behavioural response during colorectal distension (CRD) due to the inflation of a balloon introduced in the colon. This response was characterized by an elevation of the hind part of the animal body and dearly visible abdominal contraction corresponding to the severe contractions (Al Chaer, gastro 2000; Tarrerias, pain 2002; Bourdu et al., 2005). Briefly, rats were anesthetized with volatile anaesthesia (2% isoflurane), the balloon (prepared as previously described in Bourdu & al, 2005) was inserted intrarectally in a minimally invasive manner to 7 cm from the anus, and the catheter was taped to the base of the tail. After 5 minutes, rats were placed in the middle of a 40x40-cm Plexiglas box and the catheter was connected to an electronic barostat apparatus (G&J Electronics Inc., Toronto, Canada). Increasing pressure was continuously applied until pain behaviour was displayed or a cutoff pressure of 80 mm Hg was reached.

### Treatment of animals

Compounds were administrated daily by intrarectal instillations. For each enema, a catheter (2-mm Fogarty catheter) was placed in the colon at 7 cm from the anus, and the animals received 500µl of compound resuspended at optimal concentration in DMEM medium and adjusted at pH 7 by 10N NaOH if necessary for 21 days. Control animals received medium alone. Effect of the compound on visceral pain was evaluated after 2 and 3 weeks of treatment.

### Statistics

All comparisons were analyzed using the Permutation Test for two independent samples. Statistics has been calculated using the software StatXact (Cytel Inc, Cambridge, MA, USA). Differences were considered statistically significant if the P value was <0.05.

### Conclusions

The in vitro and in vivo results obtained clearly show induction of defensin expression when 5-ASA (reference compound) and R34, E1 and E2 are used. In particular and surprisingly E1 and E2 showed higher potency compared to 5-ASA.

### References

1. Barton, G.M. and Medzhitov, R. (2003) Toll-like receptor signaling pathways. Science 300 (5625), 1524-1525
2. Inohara, N. et al. (2005) NOD-LRR Proteins: Role in Host-Microbial Interactions and Inflammatory Disease. Annu. Rev. Biochem. 74, 355-383
3. Ogura, Y. et al. (2001) A frameshift mutation in NOD2 associated with susceptibility to Crohn's disease. Nature 411 (6837), 603-606
4. Hugot, J.P. et al. (2001) Association of NOD2 leucine-rich repeat variants with susceptibility to Crohn's disease. Nature 411 (6837), 599-603
5. Chamaillard, M. et al. (2003) Gene-environment interaction modulated by allelic heterogeneity in inflammatory diseases. Proc Natl Acad Sci U S A 100 (6), 3455-3460
6. Girardin, S.E. et al. (2003) Nod2 is a general sensor of peptidoglycan through muramyl dipeptide (MDP) detection. J Biol Chem 278 (11), 8869-8872
7. Inohara, N. et al. (2003) Host recognition of bacterial muramyl dipeptide mediated through NOD2. Implications for Crohn's disease. J Biol Chem 278 (8), 5509-5512
8. Kobayashi, K.S. et al. (2005) Nod2-dependent regulation of innate and adaptive immunity in the intestinal tract. Science 307 (5710), 731-734
9. Chamaillard, M. et al. (2003) An essential role for NOD1 in host recognition of bacterial peptidoglycan containing diaminopimelic acid. Nat Immunol 4 (7), 702-707
10. Girardin, S.E. et al. (2003) Nod1 detects a unique muropeptide from gram-negative bacterial peptidoglycan. Science 300 (5625), 1584-1587
11. Boughan, P.K. et al. (2006) Nucleotide-binding oligomerization domain-1 and epidermal growth factor receptor: critical regulators of beta-defensins during Helicobacter pylori infection. J Biol Chem 281 (17), 11637-11648
12. Ganz, T. (2003) Defensins: antimicrobial peptides of innate immunity. Nat Rev Immunol 3 (9), 710-720
13. Selsted, M.E. and Ouellette, A.J. (2005) Mammalian defensins in the antimicrobial immune response. Nat Immunol 6 (6), 551-557
14. Wehkamp, J. et al. (2005) Reduced Paneth cell alpha-defensins in ileal Crohn's disease. Proc Natl Acad Sci U S A 102 (50), 18129-18134
15. Ayabe, T. et al. (2000) Secretion of microbicidal alpha-defensins by intestinal Paneth cells in response to bacteria. Nat Immunol 1 (2), 113-118
16. Ghosh, D. et al. (2002) Paneth cell trypsin is the processing enzyme for human defensin-5. Nat Immunol 3 (6), 583-590
17. Wilson, C.L. et al. (1999) Regulation of intestinal alpha-defensin activation by the metalloproteinase matrilysin in innate host defense. Science 286 (5437), 113-117
18. Morrison, G. et al. (2002) Characterization of the mouse beta defensin 1, Defb1, mutant mouse model. Infect Immun 70 (6), 3053-3060
19. Sorensen, O.E. et al. (2001) Human cathelicidin, hCAP-18, is processed to the antimicrobial peptide LL-37 by extracellular cleavage with proteinase 3. Blood 97 (12), 3951-3959
20. Agerberth, B. et al. (1995) FALL-39, a putative human peptide antibiotic, is cysteine-free and expressed in bone marrow and testis. Proc Natl Acad Sci U S A 92 (1), 195-199
21. Chromek, M. et al. (2006) The antimicrobial peptide cathelicidin protects the urinary tract against invasive bacterial infection. Nat Med 12 (6), 636-641
22. Nizet, V. et al. (2001) Innate antimicrobial peptide protects the skin from invasive bacterial infection. Nature 414 (6862), 454-457
23. Rosenberger, C.M. et al. (2004) Interplay between antibacterial effectors: a macrophage antimicrobial peptide impairs intracellular Salmonella replication. Proc Natl Acad Sci U S A 101 (8), 2422-2427
24. Erdman, S. et al. (2001) Typhlocolitis in NF-kappa B-deficient mice. J Immunol 166 (3), 1443-1447
25. Rakoff-Nahoum, S. et al. (2004) Recognition of commensal microflora by toll-like receptors is required for intestinal homeostasis. Cell 118 (2), 229-241
26. Vora, P. et al. (2004) Beta-defensin-2 expression is regulated by TLR signaling in intestinal epithelial cells. J Immunol 173 (9), 5398-5405
27. Voss, E. et al. (2006) NOD2/CARD15 Mediates Induction of the Antimicrobial Peptide Human Beta-defensin-2. J Biol Chem 281 (4), 2005-2011
28. Korinek, V. et al. (1998) Depletion of epithelial stem-cell compartments in the small intestine of mice lacking Tcf-4. Nat Genet 19 (4), 379-383
29. Batlle, E. et al. (2002) Beta-catenin and TCF mediate cell positioning in the intestinal epithelium by controlling the expression of EphB/ephrinB. Cell 111 (2), 251-263
30. van Es, J.H. et al. (2005) Wnt signalling induces maturation of Paneth cells in intestinal crypts. Nat Cell Biol 7 (4), 381-386
31. Andreu, P. et al. (2005) Crypt-restricted proliferation and commitment to the Paneth cell lineage following Apc loss in the mouse intestine. Development 132 (6), 1443-1451
32. Shroyer, N.F. et al. (2005) Gfi1 functions downstream of Math1 to control intestinal secretory cell subtype allocation and differentiation. Genes Dev 19 (20), 2412-2417
33. Amann, J.M. et al. (2005) Mtgr1 is a transcriptional corepressor that is required for maintenance of the secretory cell lineage in the small intestine. Mol Cell Biol 25 (21), 9576-9585
34. Witthoft, T. et al. (2005) Enhanced human beta-defensin-2 (hBD-2) expression by corticosteroids is independent of NF-kappaB in colonic epithelial cells (CaCo2). Dig Dis Sci 50 (7), 1252-1259
35. Wang, T.T. et al. (2004) Cutting edge: 1,25-dihydroxyvitamin D3 is a direct inducer of antimicrobial peptide gene expression. J Immunol 173 (5), 2909-2912
36. Waters, W.R. et al. (2004) Mycobacterium bovis infection of vitamin D-deficient NOS2-/- mice. Microb Pathog 36 (1), 11-17
37. Liu, P.T. et al. (2006) Toll-like receptor triggering of a vitamin D-mediated human antimicrobial response. Science 311 (5768), 1770-1773
38. Shah, S. et al. (2006) The molecular basis of vitamin D receptor and beta-catenin crossregulation. Mol Cell 21 (6), 799-809
39. Peschel, A. and Sahl, H.G. (2006) The co-evolution of host cationic antimicrobial peptides and microbial resistance. Nat Rev Microbiol 4 (7), 529-536
40. Islam, D. et al. (2001) Downregulation of bactericidal peptides in enteric infections: a novel immune escape mechanism with bacterial DNA as a potential regulator. Nat Med 7 (2), 180-185
41. Salzman, N.H. et al. (2003) Enteric salmonella infection inhibits Paneth cell antimicrobial peptide expression. Infect Immun 71 (3), 1109-1115
42. Schreiber, S. et al. (2005) Genetics of Crohn disease, an archetypal inflammatory barrier disease. Nat Rev Genet 6 (5), 376-388
43. Li, J. et al. (2004) Regulation of IL-8 and IL-1{beta} expression in Crohn's disease associated NOD2/CARD15 mutations. Hum Mol Genet 13 (16), 1715-1725
44. van Heel, D.A. et al. (2005) Muramyl dipeptide and toll-like receptor sensitivity in NOD2-associated Crohn's disease. Lancet 365 (9473), 1794-1796
45. Netea, M.G. et al. (2004) NOD2 mediates anti-inflammatory signals induced by TLR2 ligands: implications for Crohn's disease. Eur J Immunol 34 (7), 2052-2059
46. Lala, S. et al. (2003) Crohn's disease and the NOD2 gene: a role for paneth cells. Gastroenterology 125 (1), 47-57
47. Ogura, Y. et al. (2003) Expression of NOD2 in Paneth cells: a possible link to Crohn's ileitis. Gut 52 (11), 1591-1597
48. Ayabe, H. et al. (2005) The Innate Intestinal Immunity By Paneth Cells and Their Alpha-Defensins in Patients With Crohn's Disease. Gastroenterology 128 (4), TI546
49. Wehkamp, J. et al. (2004) NOD2 (CARD15) mutations in Crohn's disease are associated with diminished mucosal alpha-defensin expression. Gut 53 (11), 1658-1664
50. Fellermann, K. et al. (2006) A chromosome 8 gene cluster polymorphism with low human beta-defensin 2 gene copy number predisposes to Crohn's disease of the colon. Am J Hum Genet in press
51. McGovern, D.P. et al. (2005) Association between a complex insertion/deletion polymorphism in NOD1 (CARD4) and susceptibility to inflammatory bowel disease. Hum Mol Genet 14 (10), 1245-1250
52. Viala, J. et al. (2004) Nod1 responds to peptidoglycan delivered by the Helicobacter pylori cag pathogenicity island. Nat Immunol 5 (11), 1166-1174
53. Yang, D. et al. (2004) Multiple roles of antimicrobial defensins, cathelicidins, and eosinophil-derived neurotoxin in host defense. Annu Rev Immunol 22, 181-215
54. Biragyn, A. et al. (2002) Toll-like receptor 4-dependent activation of dendritic cells by beta-defensin 2. Science 298 (5595), 1025-1029
55. Mookherjee, N. et al. (2006) Modulation of the TLR-mediated inflammatory response by the endogenous human host defense peptide LL-37. J Immunol 176 (4), 2455-2464
56. Davidson, D.J. et al. (2004) The cationic antimicrobial peptide LL-37 modulates dendritic cell differentiation and dendritic cell-induced T cell polarization. J Immunol 172 (2), 1146-1156
57. Koczulla, R. et al. (2003) An angiogenic role for the human peptide antibiotic LL-37/hCAP-18. J Clin Invest 111 (11), 1665-1672
58. Stappenbeck, T.S. et al. (2002) Developmental regulation of intestinal angiogenesis by indigenous microbes via Paneth cells. Proc Natl Acad Sci U S A 99 (24), 15451-15455
59. Darfeuille-Michaud, A. et al. (2004) High prevalence of adherent-invasive Escherichia coli associated with ileal mucosa in Crohn's disease. Gastroenterology 127 (2), 412-421
60. Chacon, O. et al. (2004) Johne's disease, inflammatory bowel disease, and Mycobacterium paratuberculosis. Annu Rev Microbiol 58, 329-363
61. Maeda, S. et al. (2005) Nod2 mutation in Crohn's disease potentiates NF-kappaB activity and IL-lbeta processing. Science 307 (5710), 734-738
62. Donnelly, J.P. et al. (2003) Antimicrobial therapy to prevent or treat oral mucositis. Lancet Infect Dis 3 (7), 405-412
63. Fahlgren, A. et al. (2004) beta-Defensin-3 and -4 in intestinal epithelial cells display increased mRNA expression in ulcerative colitis. Clin Exp Immunol 137 (2), 379-385
64. Wehkamp, J. et al. (2003) Inducible and constitutive beta-defensins are differentially expressed in Crohn's disease and ulcerative colitis. Inflamm Bowel Dis 9 (4), 215-223
65. Furrie, E. et al. (2005) Synbiotic therapy (Bifidobacterium longum/Synergy 1) initiates resolution of inflammation in patients with active ulcerative colitis: a randomised controlled pilot trial. Gut 54 (2), 242-249
66. Wehkamp, J. et al. (2004) NF-kappaB- and AP-1-mediated induction of human beta defensin-2 in intestinal epithelial cells by Escherichia coli Nissle 1917: a novel effect of a probiotic bacterium. Infect Immun 72 (10), 5750-5758
67. Dinulos J.G. et al, (2003) Keratinocyte Expression of Human β Defensin 2 following Bacterial Infection: Role in Cutaneous Host Defense. Clinical and Diagnostic Laboratory Immun (10) 161-166
68. Huang L.C. et al, (2007) Ocular surface expression and in vitro activity of antimicrobial peptides. Curr Eye Res. 32 (7-8) 595 - 609
69. Vanhinsbergh L.J. et al, (2007) Reduction of TLR2 gene expression in allergic and nonallergic rhinitis. Ann Allergy Asthma Immunol. 99(6) 509 - 16
70. Chung W.O. et al, (2007) Expression of defensins in gingiva and their role in periodontal health and disease. Curr Pharm Des. 13(30) 3073 - 83
71. Kang H.J. et al, (2006) Up-regulation of Peroxisome Proliferator-Activated Receptor {gamma} in Perennial Allergic Rhinitis. Arch Otolaryngol Head Neck Surg. 132 (11) 1196 - 1200

## Claims

1. Compound selected from 2-methoxy-3-(4'-aminophenyl)propionic acid, 5-amino-N-hydroxy-2-methoxybenzamide, 2-ethoxy-3-(4'-aminophenyl) propionic acid or 2-ethoxy-3-(3'-aminophenyl) propionic acid for use in the treatment and/or prevention of acute diverticulitis.

2. A compound of claim 1 for use according to claim 1 wherein the treatment and/or prevention of acute diverticulitis is in patients affected by colonic diverticulosis, indeterminate colitis and infectious colitis.

3. A compound of claim 1 for use according to claim 1 or claim 2 wherein the compound is 2-methoxy-3-(4'-aminophenyl)propionic acid.

4. A compound of claim 3 for use according to claim 3, wherein the compound is present in a racemic mixture or is present as the S- or R- enantiomer.

5. A compound of claim 4 for use according to claim 4 wherein the compound is present in a composition containing an excess of one enantiomer over another.

## Patentansprüche

1. Verbindung ausgewählt aus 2-Methoxy-3-(4'-aminophenyl)propansäure, 5-Amino-N-hydroxy-2-methoxybenzamid, 2-Ethoxy-3-(4'-aminophenyl)propansäure oder 2-Ethoxy-3-(3'-aminophenyl)propansäure zur Verwendung in der Behandlung und/oder Vorbeugung von akuter Divertikulitis.

2. Verbindung von Anspruch 1 zur Verwendung gemäß Anspruch 1, wobei die Behandlung und/oder Vorbeugung von akuter Divertikulitis in Patienten erfolgt, die betroffen sind von Kolondivertikulose, Colitis indeterminata und infektiöser Colitis.

3. Verbindung von Anspruch 1 zur Verwendung gemäß Anspruch 1 oder 2, wobei die Verbindung 2-Methoxy-3-(4'-aminophenyl)propansäure ist.

4. Verbindung von Anspruch 3 zur Verwendung gemäß Anspruch 3, wobei die Verbindung in einem Racematgemisch oder als das S- oder R-Enantiomer vorliegt.

5. Verbindung von Anspruch 4 zur Verwendung gemäß Anspruch 4, wobei die Verbindung in einer Zusammensetzung vorliegt, die einen Überschuss von einem Enantiomer gegenüber dem anderen enthält.

## Revendications

1. Composé choisi parmi l'acide 2-méthoxy-3-(4'-aminophényl)propionique, le 5-amino-N-hydroxy-2-méthoxybenzamide, l'acide 2-éthoxy-3-(4'-aminophényl)propionique ou l'acide 2-éthoxy-3-(3'-aminophényl)propionique pour une utilisation dans le traitement et/ou la prévention d'une diverticulite aiguë.

2. Composé de la revendication 1 pour une utilisation selon la revendication 1, où le traitement et/ou la prévention d'une diverticulite aiguë se situe chez les patients affectés par une diverticulose du côlon, une colite indéterminée et une colite infectieuse.

3. Composé de la revendication 1 pour une utilisation selon la revendication 1 ou la revendication 2, où le composé est l'acide 2-méthoxy-3-(4'-aminophényl)propionique.

4. Composé de la revendication 3 pour une utilisation selon la revendication 3, où le composé est présent dans un mélange racémique ou est présent sous la forme de l'énantiomère S ou R.

5. Composé de la revendication 4 pour une utilisation selon la revendication 4, où le composé est présent dans une composition contenant un excès d'un énantiomère par rapport à un autre.
